(19) Europäisches Patentamt
European Patent Office
Office européen des brevets

(11) EP 3 909 647 B1

(12) EUROPEAN PATENT SPECIFICATION

(45) Date of publication and mention of the grant of the patent:
**25.02.2026 Bulletin 2026/09**

(21) Application number: **19908381.7**

(22) Date of filing: **04.12.2019**

(51) International Patent Classification (IPC):
***A61K 8/02*** *(2006.01)* ***A61K 8/891*** *(2006.01)*
***A61K 8/893*** *(2006.01)* ***A61Q 17/04*** *(2006.01)*
***A61Q 19/00*** *(2006.01)* ***A61K 8/894*** *(2006.01)*

(52) Cooperative Patent Classification (CPC):
**A61K 8/891; A61K 8/022; A61K 8/893;**
A61K 2800/61

(86) International application number:
**PCT/JP2019/047308**

(87) International publication number:
**WO 2020/144978 (16.07.2020 Gazette 2020/29)**

(54) **DISPERSIBLE POWDER AND COSMETIC**

DISPERGIERBARES PULVER UND KOSMETIKUM

POUDRE DISPERSIBLE ET PRODUIT COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.01.2019 JP 2019001674**

(43) Date of publication of application:
**17.11.2021 Bulletin 2021/46**

(73) Proprietor: **Shin-Etsu Chemical Co., Ltd.**
**Tokyo 100-0005 (JP)**

(72) Inventors:
- **MORIYA, Hiroyuki**
**Annaka-shi, Gunma 379-0224 (JP)**
- **HAYAKAWA, Chihiro**
**Tokyo 100-0004 (JP)**
- **INABA, Ryuichi**
**Tokyo 100-0004 (JP)**

(74) Representative: **Wibbelmann, Jobst**
**Wuesthoff & Wuesthoff**
**Patentanwälte und Rechtsanwalt PartG mbB**
**Schweigerstrasse 2**
**81541 München (DE)**

(56) References cited:
**JP-A- 2004 300 057**
**JP-A- 2010 030 954**
**JP-A- 2013 139 400**
**JP-A- 2015 086 150**
**US-A1- 2001 007 672**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 909 647 B1

## Description

TECHNICAL FIELD

The present disclosure relates to a dispersible powder, and a cosmetic containing the dispersible powder.

BACKGROUND ART

Conventionally, studies have been conducted to improve the dispersibility of powders and for the application of such powders blended in compositions.

For example, powder-surface treatments have been studied to improve the dispersibility into aqueous media and oil materials. Patent Document 1 states that treating silsesquioxane particles with silicone improves the dispersibility into silicone oil, but the dispersibility into aqueous medium is not described. Moreover, the effect and usefulness of blending the resulting particles into a composition, particularly cosmetic composition, are not described, either.

Meanwhile, Patent Document 2 describes a method in which pigment surface is hydrophilized with a silane compound having a polyoxyalkylene group. However, this method makes the surface hydrophilicity too high, and brings about problems that the powder agglomerates, and that the tactile feel upon the application of a cosmetic blended therewith is degraded.

Further, Patent Documents 3, 4 each describe an aqueous make-up cosmetic containing a high-HLB surfactant and a powder. These are mixed in a cosmetic composition to prepare a cosmetic. However, such methods cannot suppress the agglomeration of the powder particles, and there are problems that spreadability upon application and stretchability are degraded, for example.

Patent Document 5 provides an external-use composition imparting no sticky sensation in use which contains a spherical powder of organopolysiloxane elastomer. A method of obtaining a water-in-oil emulsion-type foundation is disclosed. However, no particular information about the structure of the surface treatment agent is given, and the spherical powder of an organopolysiloxane elastomer is not surface-treated.

Patent Document 6 discloses an invention comprising a surface-treated powder which is fine particles of titanium oxide.

Patent Document 7 discloses an organopolysiloxane elastomer powder. No information about the use of a particular surface treatment agent is provided.

Patent Document 8 discloses a silicone rubber particle having a polyoxyethylene alkyl ether attached to the surface.

Patent Document 9 discloses a surface-treated powder obtained by surface-treating a powder with a crosslinked silicone elastomer. See also US 2001/007672 A1 (MIURA YOSHIMASA [JP] ET AL) 12 July 2001 (2001-07-12).

CITATION LIST

PATENT LITERATURE

Patent Document 1: Japanese Unexamined Patent Application Publication No. Hei 1-266141
Patent Document 2: Japanese Unexamined Patent Application Publication No. 2003-26958
Patent Document 3: Japanese Unexamined Patent Application Publication No. 2001-220319
Patent Document 4: Japanese Unexamined Patent Application Publication No. 2001-114649
Patent Document 5: US Unexamined Patent Application Publication No. US 2001/007672
Patent Document 6: Japanese Unexamined Patent Application Publication No. 2010-030954
Patent Document 7: Japanese Unexamined Patent Application Publication No. 2004-300057
Patent Document 8: Japanese Unexamined Patent Application Publication No. 2015-086150
Patent Document 9: Japanese Unexamined Patent Application Publication No. 2013-139400


SUMMARY OF INVENTION

TECHNICAL PROBLEM

The present invention has been made in view of the above-described problems. An object of the present invention is to provide a dispersible powder having excellent dispersibility and high dispersion stability particularly in an aqueous medium. Another object is to provide a cosmetic blended with this powder, the cosmetic being capable of forming

a uniform cosmetic film and having favorable spreadability upon application, stretchability, adhesiveness, and feeling on use as well as high stability.

SOLUTION TO PROBLEM

To achieve the object, the present invention provides a dispersible powder comprising an organopolysiloxane elastomer powder treated with a surface treatment agent, wherein

the surface treatment agent is an organopolysiloxane having a polyoxyalkylene group, a weight-average molecular weight of 300 to 300,000, and an HLB of 8 to 18, and
the organopolysiloxane having a polyoxyalkylene group has a structure as shown in the claims.

The inventive dispersible powder has excellent dispersibility and high dispersion stability particularly in an aqueous medium. Moreover, the inventive dispersible powder exhibits high dispersibility even when dispersed in a non-aqueous cosmetic.
The organopolysiloxane having a polyoxyalkylene group has a structure shown by the following general formula (1),

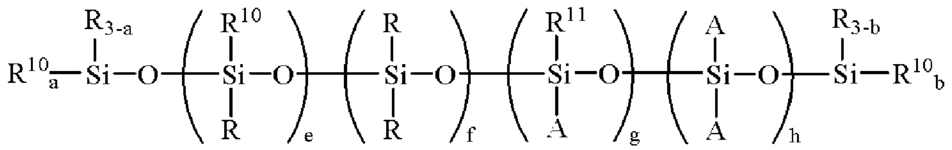
(1)

in the formula (1), each R independently represents an alkyl group having 1 to 16 carbon atoms, a fluoroalkyl group having 1 to 10 carbon atoms, or an aryl group having 6 carbon atoms;
each $R^{10}$ represents a polyoxyalkylene group shown by the following general formula (2),

$$-X-O-(C_2H_4O)_s-(C_3H_6O)_t-R^{12} \qquad (2)$$

in the formula (2), X represents a divalent hydrocarbon group having 1 to 10 carbon atoms, "s" represents an integer of 1 to 100, "t" represents an integer of 0 to 50, and $R^{12}$ represents a hydrogen atom, a monovalent hydrocarbon group having 1 to 6 carbon atoms, or a monovalent acyl group having 1 to 6 carbon atoms;

$R^{11}$ represents an organic group selected from $R^{10}$ or R; and
"A" represents an organopolysiloxane segment shown by the following general formula (3) or (4),

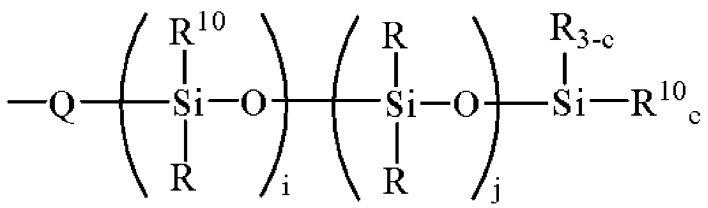
(3)

$$-X-Si(OSi(CH_3)_3)_3 \qquad (4)$$

in the general formula (3), R and $R^{10}$ are as defined above, and Q represents an oxygen atom or a divalent hydrocarbon group having 1 to 3 carbon atoms; in the general formula (4), X is as defined above, and
in the general formulae (1) and (3), "a", "b", and "c" each independently represent an integer of 0 to 3, "e" represents an integer of 0 to 500, "f" represents an integer of 0 to 50,000, "g" represents an integer of 0 or 1, "h" represents an integer of 0 or 1, "i" represents an integer of 0 to 500, and "j" represents an integer of 0 to 10,000, provided that when $R^{11}$ is $R^{10}$, $1 \leq a+b+c+e+g+i$, and when $R^{11}$ is R, $1 \leq a+b+c+e+i$; and at least one $R^{10}$ is present in the general formula (1).
When such a surface treatment agent is used, a powder having more excellent dispersibility is obtained.
Additionally, the present invention provides a cosmetic comprising the above-described dispersible powder.
Such a cosmetic is excellent in dispersibility, high in dispersion stability, and capable of forming a uniform cosmetic film. The cosmetic also has favorable spreadability upon application, stretchability, adhesiveness, and feeling on

use.

Preferably, the cosmetic further comprise water.

Thereby, the cosmetic can have favorable feeling on use, and excellent usability and persistency.

More preferably, the cosmetic further comprises a hydrophilic substance selected from propylene glycol, trimethylene glycol, dipropylene glycol, 1,3-butylene glycol, methylpropanediol, pentylene glycol, glycerin, diglycerin, ethylhexylglycerin, triglycerin, polyglycerin, and ethanol.

This can make the cosmetic have more favorable feeling on use and excellent usability and persistency.

## ADVANTAGEOUS EFFECTS OF INVENTION

As described above, the inventive dispersible powder has excellent dispersibility and high dispersion stability, is capable of forming a uniform cosmetic film when blended in a cosmetic, and results in favorable spreadability upon application, stretchability, adhesiveness, and feeling on use.

## DESCRIPTION OF EMBODIMENTS

As noted above, there have been demands for a dispersive powder treated to be excellent in dispersibility, high in dispersion stability, and capable of forming a uniform cosmetic film when blended in a cosmetic, thereby the cosmetic having favorable spreadability upon application, stretchability, adhesiveness, and feeling on use.

The present inventors have earnestly studied to achieve the above-described objects and consequently found that an organopolysiloxane elastomer powder surface-treated with a polyoxyalkylene group-containing organopolysiloxane having specific molecular weight and hydrophilicity-hydrophobicity balance has excellent dispersibility. Additionally, no such dispersible powders treated with the surface treatment agent have been added to cosmetics.

Furthermore, the inventors have found that a cosmetic blended with the inventive dispersible powder has favorable spreadability upon application and favorable stretchability, adhesiveness, and feeling on use. These findings have led to the present invention.

Specifically, the present invention is a dispersible powder comprising an organopolysiloxane elastomer powder treated with a surface treatment agent, wherein

> the surface treatment agent is an organopolysiloxane having a polyoxyalkylene group, a weight-average molecular weight of 300 to 300,000, and an HLB of 8 to 18, and
> the organopolysiloxane having a polyoxyalkylene group has a structure as shown in the claims.

In addition, the present invention provides a cosmetic comprising the dispersible powder.

Hereinafter, the present invention will be described in detail, but the present invention is not limited thereto.

### [Dispersible Powder]

The inventive dispersible powder is an organopolysiloxane elastomer powder treated with a surface treatment agent. Hereinbelow, the inventive dispersible powder will be described.

### [Powder]

Examples of the organopolysiloxane elastomer powder used in the present invention include (dimethicone/vinyl dimethicone) crosspolymer, (dimethicone/phenyl vinyl dimethicone) crosspolymer, (vinyl dimethicone/lauryl dimethicone) crosspolymer, (vinyl dimethicone/methicone silsesquioxane) crosspolymer, (diphenyl dimethicone/vinyl diphenyl dimethicone/silsesquioxane) crosspolymer, polysilicone-1 crosspolymer, polysilicone-22, and (PEG-15/lauryl dimethicone) crosspolymer.

The powder is preferably (dimethicone/vinyl dimethicone) crosspolymer, (dimethicone/phenyl vinyl dimethicone) crosspolymer, (vinyl dimethicone/lauryl dimethicone) crosspolymer, (vinyl dimethicone/methicone silsesquioxane) crosspolymer, (diphenyl dimethicone/vinyl diphenyl dimethicone/silsesquioxane) crosspolymer, polysilicone-1 crosspolymer, or polysilicone-22, further preferably (dimethicone/vinyl dimethicone) crosspolymer or (vinyl dimethicone/methicone silsesquioxane) crosspolymer.

The particle shape of the organopolysiloxane elastomer powder may be spherical, irregular, concaved, or cup-like shape.

The organopolysiloxane elastomer powder has an average particle diameter of preferably 0.5 to 100 μm, more preferably 1 to 50 μm. Note that, unless otherwise particularly specified, the average particle diameter according to the present invention refers to a volume average particle diameter that can be measured through observation with an optical

microscope or scanning electron microscope. With the average particle diameter in the above-described ranges, a cosmetic containing the inventive dispersible powder has more favorable stretchability, and a cosmetic film formed by applying and spreading the cosmetic has more favorable uniformity.

[Surface Treatment Agent]

The inventive dispersible powder is an organopolysiloxane elastomer powder treated with a surface treatment agent as described above. The present invention is characterized by using a specific organopolysiloxane as the surface treatment agent.

The surface treatment agent is an organopolysiloxane having a polyoxyalkylene group, a weight-average molecular weight of 300 to 300,000, and an HLB of 8 to 18. The weight-average molecular weight of the organopolysiloxane is preferably 300 to 250,000, further preferably 500 to 100,000. If the weight-average molecular weight is less than 300, favorable dispersibility in a cosmetic may not be exhibited in some cases. Meanwhile, if the weight-average molecular weight is more than 300,000, stickiness or heaviness may be perceived in some cases. Note that, in the present invention, the weight-average molecular weight can be determined by gel permeation chromatography (GPC) analysis as weight-average molecular weight in terms of polystyrene (hereinafter the same).

The HLB of the surface treatment agent is 8 to 18, more preferably 8 to 16. If the HLB is less than 8, the affinity between the dispersible powder and an aqueous medium is so low that the dispersion is hindered in some cases. Meanwhile, if the HLB is more than 18, the organopolysiloxane having a polyoxyalkylene group is considerably separated from the dispersible powder, so that the dispersion of the dispersible powder into an aqueous medium is hindered in some cases.

Note that the HLB value in the present invention is a value calculated according to the Griffin equation shown by the following equation:

HLB = [Molecular weight of polyoxyalkylene moiety/Molecular weight of organopolysiloxane having a polyoxyalkylene group] $\times$ 20.

When two or more kinds of organopolysiloxane having a polyoxyalkylene group which have different HLB values are used in combination, the HLB is an average by mass.

The organopolysiloxane having a polyoxyalkylene group used as the surface treatment agent in the present invention has a structure shown by the following general formula (1).

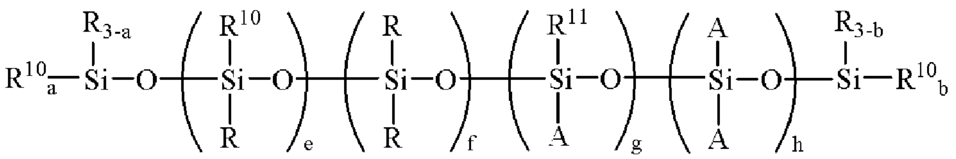
(1)

In the formula (1), each R is independently an alkyl group having 1 to 16 carbon atoms, a fluoroalkyl group having 1 to 10 carbon atoms, or an aryl group having 6 carbon atoms, most preferably a methyl group.

Each $R^{10}$ is a polyoxyalkylene group shown by the following general formula (2).

$$X\text{-}O(C_2H_4O)_s\text{-}(C_3H_6O)_t\text{-}R^{12} \qquad (2)$$

In the formula (2), X is a divalent hydrocarbon group having 1 to 10 carbon atoms, preferably a divalent hydrocarbon group having 2 to 8 carbon atoms, most preferably a propylene group or an isobutylene group. "s" is an integer of 1 to 100, preferably 4 to 20. "t" is an integer of 0 to 50, preferably 0 to 20. $R^{12}$ is a hydrogen atom, a monovalent hydrocarbon group having 1 to 6 carbon atoms, or a monovalent acyl group having 1 to 6 carbon atoms, preferably a hydrogen atom, a methyl group, or an acetyl group.

$R^{11}$ is an organic group selected from $R^{10}$ or R.

"A" is an organopolysiloxane segment shown by the following general formula (3) or (4).

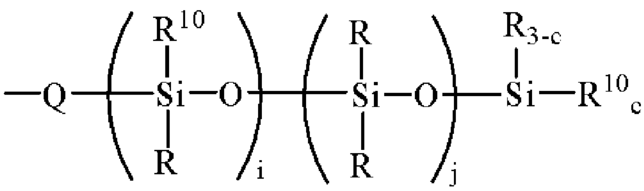
(3)

$$-X-Si(OSi(CH_3)_3)_3 \quad (4)$$

In the general formula (3), R and $R^{10}$ are as defined above, and Q is an oxygen atom or a divalent hydrocarbon group having 1 to 3 carbon atoms, preferably an oxygen atom or an ethylene group. In the general formula (4), X is as defined above.

In the general formulae (1) and (3), "a", "b", and "c" are each independently an integer of 0 to 3. "e" is an integer of 0 to 500, preferably 1 to 100, more preferably 4 to 50. "f" is an integer of 0 to 50,000, preferably 0 to 10,000. "g" is an integer of 0 or 1. "h" is an integer of 0 or 1. "i" is an integer of 0 to 500, preferably 1 to 100. "j" is an integer of 0 to 10,000, preferably 0 to 100. Note that when $R^{11}$ is $R^{10}$, $1 \leq a+b+c+e+g+I$; when $R^{11}$ is R, $1 \leq a+b+c+e+i$. At least one $R^{10}$ is present in the general formula (1).

[Method for Producing Dispersible Powder]

The method for producing the inventive dispersible powder is a method in which the organopolysiloxane elastomer powder is treated with the surface treatment agent to produce a dispersible powder, and may include conducting drying process, wetting process, or the like. As the equipment, a bead mill, a hammer mill, a Nauta mixer, or the like can be used. Heating can also be performed during the surface treatment, and the temperature is preferably 30 to 150°C, particularly preferably 30 to 110°C. Particularly, heating the organopolysiloxane surface treatment agent (surfactant) lowers the viscosity, causing favorable adsorption to the powder surface, and suppressing the agglomeration. Accordingly, it is preferable to perform the heating treatment. Additionally, during the heat treatment, re-attachment to the powder surface may occur via cleavage of siloxane bond in the organopolysiloxane surfactant, or via Si-H group and/or alkoxysilyl group remaining in slight amount in the organopolysiloxane. Note that, during the treatment with the surface treatment agent, an optional component, such as water and an alcohol, can be added as necessary. To obtain a desired dispersible powder, such operations as stirring, pulverization, drying, etc. can also be performed as necessary.

[Cosmetic]

The present invention also provides a cosmetic containing the inventive dispersible powder (hereinafter also referred to as "the inventive cosmetic"). This cosmetic can be blended with a medium that is allowable for cosmetics.

The inventive dispersible powder is contained in an amount of preferably 0.1 to 90 mass%, more preferably 0.2 to 50 mass%, further preferably 0.5 to 20 mass%, relative to the whole cosmetic. When the content of the dispersible powder is within these ranges, the softness and stretchability of the elastomer itself can be felt, and the resulting cosmetic has high stability without powdery feel.

More preferably, the inventive cosmetic further contains water, besides the dispersible powder. This enables the inventive cosmetic to have favorable feeling on use and excellent usability and persistency.

The water content is preferably 1 to 98 mass%, more preferably 2 to 90 mass%, further preferably 3 to 80 mass%, of the whole cosmetic. When the water content is within these ranges, a cosmetic with favorable stability and usability is obtained.

More preferably, the inventive cosmetic further contains, besides the dispersible powder and water, a hydrophilic substance selected from propylene glycol, trimethylene glycol, dipropylene glycol, 1,3-butylene glycol, methylpropanediol, pentylene glycol, glycerin, diglycerin, ethylhexylglycerin, triglycerin, polyglycerin, and ethanol. Thereby, the inventive cosmetic has favorable feeling on use and excellent usability and persistency.

These hydrophilic substances are contained in an amount of preferably 1 to 80 mass%, more preferably 1 to 40 mass%, further preferably 3 to 30 mass%, relative to the whole cosmetic. When the content of the hydrophilic substance is within these ranges, a cosmetic with better stability and usability is obtained.

The inventive cosmetic may contain a volatile silicone oil or a volatile organic oil. Examples of the volatile silicone oil include dimethylpolysiloxanes (dimer, trimer, tetramer, pentamer), caprylyl methicone, octamethylcyclotetrasiloxane, decamethylcyclopentasiloxane, dodecamethylcyclohexasiloxane, tetramethyltetrahydrogencyclotetrasiloxane, tristrimethylsiloxymethylsilane, tetrakistrimethylsiloxysilane, etc.

The volatile organic oil may be a hydrocarbon oil or ester. Examples thereof include α-olefin oligomer, light isoparaffin, isododecane, light liquid isoparaffin, butyl acetate, etc. Preferable examples are dimethylpolysiloxanes (tetramer, pentamer), caprylyl methicone, tristrimethylsiloxymethylsilane, tetrakistrimethylsiloxysilane, light isoparaffin, isododecane, light liquid isoparaffin, and butyl acetate. These volatile oil materials are contained in an amount of preferably 1 to 98 mass%, more preferably 1 to 50 mass%, further preferably 3 to 30 mass%, relative to the whole cosmetic.

In addition to the volatile oil(s), the inventive cosmetic can further contain an oil material that is allowable for cosmetics. Examples of such a liquid oil material can include one or more kinds of silicone oil, hydrocarbon oil, higher fatty acid, higher alcohol, polar oils, such as ester oil, glyceride oil, and natural animal and vegetable oils, semisynthetic oil,

and/or fluorinated oil, etc.

Examples of the silicone oil include linear or branched organopolysiloxanes having low viscosity to high viscosity, such as nonvolatile dimethylpolysiloxane, phenyltrimethicone, methylphenylpolysiloxane, dimethylsiloxy phenyl trimethicone, diphenylsiloxy phenyl trimethicone (for example, KF-56A manufactured by Shin-Etsu Chemical Co., Ltd.), methylhexylpolysiloxane, methylhydrogenpolysiloxane, and dimethylsiloxane/methylphenylsiloxane copolymer; tetramethyltetraphenylcyclotetrasiloxane, amino-modified organopolysiloxane; silicone rubbers, such as highly polymerized gummy dimethylpolysiloxane, gummy amino-modified organopolysiloxane, and gummy dimethylsiloxane/methylphenylsiloxane copolymer; solutions of silicone gum or rubber in cyclic siloxane; trimethylsiloxy silicic acid, and solutions of trimethylsiloxy silicic acid in cyclic organopolysiloxane; higher alkoxy-modified organopolysiloxanes, such as stearoxy silicone; higher fatty acid-modified organopolysiloxane, alkyl-modified organopolysiloxane, long chain alkyl-modified organopolysiloxane, fluorine-modified organopolysiloxane, silicone resin, silicone resin solutions, etc.

Examples of the hydrocarbon oil include nonvolatile ones, such as ozocerite, squalane, synthetic squalane, vegetable squalane, squalene, ceresin, paraffin, paraffin wax, polyethylene wax, polyethylene/polypropylene wax, (ethylene/propylene/styrene)copolymer, (butylene/propylene/styrene)copolymer, liquid paraffin, liquid isoparaffin, pristane, polyisobutylene, hydrogenated polyisobutene, microcrystalline wax, vaseline, etc.

Examples of the higher fatty acid include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, 12-hydroxystearic acid, etc. Examples of the higher alcohol include lauryl alcohol, myristyl alcohol, palmityl alcohol, stearyl alcohol, behenyl alcohol, hexadecyl alcohol, oleyl alcohol, isostearyl alcohol, hexyl dodecanol, octyl dodecanol, cetostearyl alcohol, 2-decyl tetradecynol, cholesterol, phytosterol, POE cholesterol ether, monostearyl glycerin ether (batyl alcohol), monooleyl glyceryl ether (selachyl alcohol), etc.

Examples of the ester oil include diisobutyl adipate, 2-hexyldecyl adipate, di-2-heptylundecyl adipate, N-alkylglycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyl dodecyl gum ester, oleyl oleate, octyldodecyl oleate, decyl oleate, neopentyl glycol dioctanoate, neopentyl glycol dicaprate, coco-(caprate/caprylate), triethyl citrate, 2-ethylhexyl succinate, isocetyl stearate, butyl stearate, diisopropyl sebacate, di-2-ethylhexyl sebacate, cetyl lactate, myristyl lactate, isononyl isononanoate, isotridecyl isononanoate, isopropyl palmitate, 2-ethylhexyl palmitate, 2-hexyldecyl palmitate, 2-heptylundecyl palmitate, cholesteryl 12-hydroxystearate, dipentaerythritol fatty acid ester, isopropyl myristate, octyldodecyl myristate, 2-hexyldecyl myristate, myristyl myristate, hexyldecyl dimethyloctanoate, ethyl laurate, hexyl laurate, N-lauroyl-L-glutamic acid-2-octyldodecyl ester, lauroyl sarcosine isopropyl ester, diisostearyl malate, etc. Examples of the glyceride oil include acetoglyceryl, glyceryl triisooctanoate, glyceryl triisostearate, glyceryl triisopalmitate, glyceryl monostearate, glyceryl di-2-heptylundecanoate, glyceryl trimyristate, diglyceryl myristate isostearate, glyceryl triethylhexanoate, etc.

Examples of the natural animal and vegetable oils and semisynthetic oils include avocado oil, linseed oil, almond oil, Chinese wax, perilla oil, olive oil, cacao butter, kapok wax, kaya oil, carnauba wax, cod-liver oil, candelilla wax, purified candelilla wax, beef tallow, neats-foot oil, beef bone fat, cured beef tallow, apricot kernel oil, whale wax, hydrogenated oil, wheat germ oil, sesame oil, rice germ oil, rice bran oil, sugarcane wax, sasanqua oil, safflower oil, shea butter, Chinese tung oil, cinnamon oil, jojoba wax, squalane, squalene, shellac wax, turtle oil, soybean oil, tea seed oil, camellia oil, evening primrose oil, corn oil, pig fat, rapeseed oil, Japanese tung oil, bran wax, germ oil, horse fat, persic oil, palm oil, palm kernel oil, castor oil, cured castor oil, methyl ester of castor oil fatty acid, sunflower oil, grape seed oil, bayberry wax, jojoba oil, hydrogenated jojoba oil, macademia nut oil, bees wax, mink oil, meadowfoam seed oil, cotton seed oil, cotton wax, Japan wax, Japan wax kernel oil, montan wax, coconut oil, cured coconut oil, tri-coconut fatty acid glyceride, mutton tallow, peanut oil, lanolin, liquid lanolin, reduced lanolin, lanolin alcohol, hard lanolin, lanolin acetate, lanolin alcohol acetate, isopropyl lanolin fatty acid, egg-yolk oil, etc.

Examples of the fluorinated oil material include perfluoro polyether, perfluoro decalin, perfluoro octane, etc.

In the present invention, the oil material that is allowable for cosmetics, other than the volatile oil, is blended in an amount of suitably 1 to 98 mass%, preferably 1 to 50 mass%, further preferably 3 to 30 mass%, relative to the whole cosmetic, although the amount varies depending on the form of the cosmetic.

The inventive cosmetic can further contain one or more kinds of ultraviolet (UV) absorber and/or UV-scattering agent. This makes the inventive cosmetic not only have favorable feeling on use and excellent usability and persistency, but also capable of blocking ultraviolet ray.

Examples of the UV-absorber include benzoic acid UV-absorbers, such as para-amino benzoic acid; anthranilic acid UV-absorbers, such as methyl anthranilate; salicylic acid UV-absorbers, such as methyl salicylate; cinnamic acid UV-absorbers, such as ethylhexyl para-methoxycinnamate; benzophenone UV-absorbers, such as 2,4-dihydroxybenzophenone; urocanic acid UV-absorbers, such as ethyl urocanate; triazine UV-absorbers, such as bis-ethylhexyloxyphenol methoxyphenyl triazine; dibenzoylmethane UV-absorbers, such as 4-t-butyl-4'-methoxy-dibenzoylmethane; etc. It is also possible to use silicone derivatives having an UV-absorbing functional group described above.

Examples of the UV-scattering agent include particles that absorb and scatter ultraviolet light, such as titanium

oxide microparticles, iron-containing titanium oxide microparticles, zinc oxide microparticles, cerium oxide microparticles, and composites thereof. Among them, preferable are cinnamic acid UV-absorbers, dibenzoylmethane UV-absorbers, titanium oxide microparticles (for example, SPD-T5 manufactured by Shin-Etsu Chemical Co., Ltd.), and zinc oxide microparticles (for example, SPD-Z5 manufactured by Shin-Etsu Chemical Co., Ltd.; etc.).

The inventive cosmetic can contain one or more kinds of surfactant. Thereby, the inventive cosmetic is allowed to be more excellent in emulsification stability and usability depending on the usage. The surfactant includes anionic, cationic, nonionic, and amphoteric surfactants. The cosmetic can employ and contain, without particular limitation, any surfactant that is used for ordinal cosmetics.

Specific examples of the anionic surfactant include fatty acid soap, such as sodium stearate and triethanolamine palmitate; alkyl ether carboxylic acids and salts thereof; condensation salts of amino acid and fatty acid; alkane sulfonate salts; alkene sulfonate salts; sulfonate salts of fatty acid ester, sulfonate salts of fatty acid amide; sulfonate salts of formalin condensate; alkyl sulfate ester salts; sulfate ester salts of secondary higher alcohols; sulfate ester salts of alkyl and allyl ether; sulfate ester salts of fatty acid ester; sulfate ester salts of fatty acid alkylolamide; sulfate ester salts, such as Turkey red oil; alkyl phosphate salts; ether phosphate salts; alkyl allyl ether phosphate salts amide phosphate salts; N-acyl lactate salt; N-acylsarcosine salts; N-acylamino acid activators; carboxy vinyl polymers; polyacryl amide-based anionic surfactants; etc. Examples of the cationic surfactant include alkyl amine salts; amine salts of polyamines, amino alcohol fatty acid derivatives, etc.; alkyl quaternary ammonium salts (for example, behentrimonium chloride), aromatic quaternary ammonium salts, pyridinium salts, imidazolium salts, etc.

Examples of the nonionic surfactant include sorbitan fatty acid esters, glycerin fatty acid esters, polyglycerin fatty acid esters, propylene glycol fatty acid esters, polyethylene glycol fatty acid esters, sucrose fatty acid esters, methyl glucoside fatty acid esters, alkyl polyglucosides, polyoxyethylene alkyl ethers, polyoxypropylene alkyl ethers, polyoxyethylene alkyl phenyl ethers, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanol ethers, polyoxyethylene phytosterol ethers, polyoxyethylene cholestanol ethers, and polyoxyethylene cholesterol ethers;
linear or branched polyoxyalkylene-modified organopolysiloxanes (for example, KF-6017, KF-6017P, KF-6028 manufactured by Shin-Etsu Chemical Co., Ltd.), linear or branched organopolysiloxanes co-modified with polyoxyalkylene and alkyl (for example, KF-6038, KF-6048 manufactured by Shin-Etsu Chemical Co., Ltd.; etc.), linear or branched polyglycerin-modified organopolysiloxanes (for example, KF-6100, KF-6104, KF-6106 manufactured by Shin-Etsu Chemical Co., Ltd.; etc.), linear or branched organopolysiloxanes co-modified with polyglycerin and alkyl (for example, KF-6105 manufactured by Shin-Etsu Chemical Co., Ltd.; etc.), alkanol amides, sugar ethers, sugar amides, etc.

Examples of the amphoteric surfactant include betaines, aminocarboxylic acid salts, imidazoline derivatives, amide amine type, etc.

The inventive cosmetic may further contain a composition composed of liquid oil material and one or more kinds of crosslinked organopolysiloxane polymer having no hydrophilic group. The crosslinked organopolysiloxane polymer can be obtained by reaction of alkylhydrogenpolysiloxane and/or arylhydrogenpolysiloxane with a crosslinking agent that has a reactive vinyl type unsaturated group(s) at the terminal of the molecular chain.

Examples of the alkylhydrogenpolysiloxane can include linear or partially branched methylhydrogenpolysiloxane, methylhydrogenpolysiloxane in which an alkyl chain with 6 to 20 carbon atoms is grafted, etc. Each of these molecules has to contain two or more hydrogen atoms that are bonded to silicon atoms in average. Examples of the crosslinking agent include a molecule having two or more vinyl type reaction moieties, such as methylvinylpolysiloxane and $\alpha,\omega$-alkenyldiene.

Examples thereof include compositions described in JP 1925781B, JP 1932769B, WO 03/24413A1, and JP 2009-185296A. Such crosslinked methylpolysiloxane is swollen with low viscosity silicone with the viscosity of 0.65 to 100.0 $mm^2$/second (at 25°C), hydrocarbon oil, such as liquid paraffin, squalane, and isododecane, glyceride oil, such as trioctanoin, and/or ester oil in an amount of the own weight or more, for example. Examples of commercially products of these crosslinked organopolysiloxanes are not particularly limited to, but include KSG-15, KSG-16, KSG-18, KSG-18A, KSG-1610, and USG-103, which are pastes mixed with silicone oil; USG-106, KSG-41, KSG-42, KSG-43, KSG-44, and KSG-810, which are pastes mixed with hydrocarbon oil or triglyceride oil (all of which are manufactured by Shin-Etsu Chemical Co., Ltd.); etc.

The composition composed of liquid oil material and the crosslinked organopolysiloxane having no hydrophilic group is blended in an amount of preferably 0.1 to 50 mass%, further preferably 1 to 30 mass%, relative to the whole amount of the cosmetic.

The inventive cosmetic may further contain a composition composed of liquid oil material and one or more kinds of crosslinked organopolysiloxane polymer having a hydrophilic group. The hydrophilic group is preferably a polyether group and a polyglycerin group. The crosslinked organopolysiloxane polymer having a polyether group and/or a polyglycerin group can be obtained by reaction of alkylhydrogenpolysiloxane and a crosslinking agent that has a reactive vinyl type unsaturated group(s) at the terminal of the molecular chain. Examples of the alkylhydrogenpolysiloxane can

include methylhydrogenpolysiloxane in which a polyoxyethylene chain is grafted, methylhydrogenpolysiloxane in which a polyglycerin chain is grafted, etc. Each of these molecules has to contain two or more hydrogen atoms that are bonded to silicon atoms in average.

This crosslinked organopolysiloxane polymer is swollen with low viscosity silicone with the viscosity of 0.65 to 100.0 $mm^2$/second (at 25°C), hydrocarbon oil, such as liquid paraffin, squalane, and isododecane, glyceride oil, such as trioctanoin, and/or ester oil in an amount of the own weight or more. Examples of the crosslinking agent include a molecule having two or more vinyl type reaction moieties, such as methylvinylpolysiloxane, $\alpha,\omega$-alkenyldiene, glycerin triallyl ether, polyoxyalkynylated glycerin triallyl ether, trimethylolpropane triallyl ether, and polyoxyalkynylated trimethylolpropane triallyl ether, provided that the crosslinked product by the reaction therewith contains at least one hydrophilic group.

Preferable examples of the composition include ones described in JP 2631772B, JP H09-136813A1, JP 2001-342255A1, WO 03/20828A1, and JP 2009-185296A. Examples of commercially products of these crosslinked organopolysiloxanes are not particularly limited to, but include KSG-210, KSG-240, and KSG-710, which are pastes mixed with silicone oil; KSG-310, KSG-320, KSG-330, KSG-340, KSG-820, KSG-830, KSG-840, and KSG-850Z which are pastes mixed with hydrocarbon oil or triglyceride oil (all of which are manufactured by Shin-Etsu Chemical Co., Ltd.); etc.

The composition composed of liquid oil material and the crosslinked organopolysiloxane having a hydrophilic group(s) is blended in an amount of preferably 0.1 to 50 mass%, further preferably 1 to 30 mass%, relative to the whole amount of the cosmetic.

The inventive cosmetic may contain silicone wax in accordance with the object. This silicone wax is preferably polylactone-modified polysiloxane having bonded polylactone, which is a ring opening polymerization product of a lactone compound having a ring of five or more atoms. Alternatively, this silicone wax is preferably acrylic-modified polysiloxane with the molecule containing at least one functional group selected from a pyrrolidone group, a long-chain alkyl group, a polyoxyalkylene group, a fluoroalkyl group, and anionic groups, such as a carboxylic acid. Examples of commercial products of wax having a long-chain alkyl group include KP-561P and KP-562P (all of which are manufactured by Shin-Etsu Chemical Co., Ltd.), etc.

The inventive cosmetic may further contain a component(s) used for ordinary cosmetics, an oil-soluble gelation agent (organic-modified clay mineral), various powders, antiperspirant, moisturizer, antimicrobial preservative, fragrance, salt, antioxidant, pH adjuster, chelating agent, cooling agent, anti-inflammatory agent, skin care component (such as whitening agent, cell activator, rough skin improver, blood circulation promoter, skin astringent, antiseborrheic agent), vitamin, amino acid, nucleic acid, hormone, inclusion compound, etc.

Examples of the oil-soluble gelation agent include one or more kinds of oil-soluble gelation agents selected from metal soap, such as aluminum stearate, magnesium stearate, and zinc myristate; amino acid derivatives, such as N-lauroyl-L-glutamic acid and $\alpha,\gamma$-di-n-butyl amine; dextrin fatty acid esters, such as dextrin palmitate ester, dextrin stearate ester, and dextrin 2-ethylhexanoate palmitate ester; sucrose fatty acid esters, such as sucrose palmitate ester and sucrose stearate ester; fructo-oligosaccharide fatty acid esters, such as fructo-oligosaccharide stearate ester and fructo-oligosaccharide 2-ethylhexanoate ester; benzylidene derivatives of sorbitol, such as monobenzylidene sorbitol and dibenzylidene sorbitol; organic-modified clay mineral, such as dimethyl benzyl dodecyl ammonium montmorillonite clay and dimethyl dioctadecyl ammonium hectorite; etc.

Examples of the inorganic powder include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, lithia mica, silicic acid, anhydrous silicic acid, aluminum silicate, magnesium silicate, aluminum magnesium silicate, calcium silicate, barium silicate, strontium silicate, metal tungstate salt, hydroxy apatite, vermiculite, higilite, bentonite, montmorillonite, hectorite, zeolite, ceramic powder, dibasic calcium phosphate, alumina, aluminum hydroxide, boron nitride, boron nitride, silica, and silica silylate.

Examples of the color pigment include inorganic red pigment, such as iron oxide, iron hydroxide, and iron titanate; inorganic brown pigment, such as $\gamma$-iron oxide; inorganic yellow pigment, such as yellow iron oxide and loess; inorganic black pigment, such as black iron oxide and carbon black; inorganic purple pigment, such as manganese violet and cobalt violet; inorganic green pigment, such as chromium hydroxide, chromium oxide, cobalt oxide, and cobalt titanate; inorganic blue pigment, such as Prussian blue and ultramarine blue; laked tar dye; laked natural dye; synthetic resin powder pigment obtained by hybridization of these powders; etc.

Examples of the pearl pigment include muscovite coated with titanium oxide, mica coated with titanium oxide, oxychloro bismuth, oxychloro bismuth coated with titanium oxide, talc coated with titanium oxide, fish scale foil, color mica coated with titanium oxide, etc. Examples of the metal powder pigment include aluminum powder, copper powder, stainless powder, etc.

The powders that can be added to the inventive cosmetic preferably have hydrophobic surface. The term hydrophobic means that the powder does not disperse in water. If the powder surface is hydrophobic from the beginning, the powder may be used as it is. As necessary, a powder may be hydrophobized as follows and used. The use of such hydrophobic powder improves the uniformity of the cosmetic film, settlement upon application, and adhesiveness. In addition, each of the powders preferably has an average particle diameter of 200 μm or less. The particles constituting the

powders are not particularly limited in regard to the form (spherical, needle-like, plate-like, etc.) or particle structure (porous, non-porous, etc.). The average particle diameter is preferably 200 μm or less, more preferably 100 μm or less, further preferably 50 μm or less. Here, the average particle diameter refers to volume-average particle diameter and can be measured by laser scattering method etc.

The powders are preferably surface-hydrophobized with a treatment agent selected from silicone, fluorine compounds, silane coupling agents, titanium coupling agents, N-acylated amino acids, and metal soaps. Particularly, since the inorganic powder, color pigment, and pearl pigment have hydrophilic surfaces, these are preferably used after subjected to the surface hydrophobic treatment with the treatment agent selected from silicone, fluorine compounds, silane coupling agents, titanium coupling agents, N-acylated amino acids, and metal soaps.

Examples of the antiperspirant include aluminum chlorohydrate, aluminum chloride, aluminum sesquichlorohydrate, zirconyl hydroxychloride, aluminum zirconium hydroxychloride, aluminum zirconium glycine complex, etc.

Examples of the moisturizer include sorbitol, xylitol, maltitol, polyethylene glycol, polysaccharides, hydroxyethylcellulose, xanthan gum, hyaluronic acid, chondroitin sulfate, pyrrolidone carboxylate, polyoxyethylene methyl glucoside, polyoxypropylene methyl glucoside, egg-yolk lecithin, soybean lecithin, phosphatidylcholine, phosphatidylethanolamine, phosphatidylserine, phosphatidylglycerol, phosphatidylinositol, sphingophospholipid, etc.

Examples of the antimicrobial preservative include alkyl para-oxybenzoate (paraben), benzoic acid, sodium benzoate, sorbic acid, potassium sorbate, phenoxy ethanol, etc. Examples of the antimicrobial include benzoic acid, salicylic acid, carbolic acid, sorbic acid, alkyl para-oxybenzoate, p-chloro-m-cresol, hexachlorophene, benzalkonium chloride, chlorhexidine chloride, trichlorocarbanilide, photosensitizers, phenoxy ethanol, etc.

The fragrance includes natural fragrance and synthetic fragrance. Examples of the natural fragrance include vegetable fragrances isolated from flower, leaf, material, peel, etc.; and animal fragrances, such as musk (bisabolol) and civet. Examples of the synthetic fragrance include hydrocarbons, such as monoterpene; alcohols, such as aliphatic alcohols and aromatic alcohols; aldehydes, such as terpene aldehyde and aromatic aldehyde; ketones, such as alicyclic ketones; esters, such as terpene-based esters; lactones; phenols; oxides; nitrogen-containing compounds; acetals; etc.

Examples of the salt include salts, such as inorganic salts, organic acid salts, amine salts, and amino acid salts. Examples of the inorganic salts include sodium salts, potassium salts, magnesium salts, calcium salts, aluminum salts, zirconium salts, barium salts, zinc salts, and the like of inorganic acid, such as hydrochloric acid, sulfuric acid, carbonic acid, and nitric acid. Examples of the organic acid salts include salts of organic acids, such as acetic acid, dehydroacetic acid, citric acid, malic acid, succinic acid, ascorbic acid, and stearic acid. Examples of the amine salts and the amino acid salts include salts of amines, such as triethanolamine; salts of amino acids, such as glutamic acid; etc. Furthermore, it is also possible to use salts of hyaluronic acid, chondroitin sulfate; aluminum zirconium glycine complex; acid-alkali neutral salts, which are used in cosmetic preparations; etc.

Examples of the antioxidant include tocopherol, p-t-butylphenol, butylhydroxyanisol, dibutylhydroxytoluene (BHT), phytic acid, etc.

Examples of the pH adjuster include lactic acid, citric acid, glycolic acid, succinic acid, tartaric acid, dl-malic acid, potassium carbonate, sodium bicarbonate, ammonium bicarbonate, etc.

Examples of the chelating agent include alanine, sodium edetate, sodium polyphosphate, sodium metaphosphate, phosphoric acid, etc.

Examples of the cooling agent include L-menthol, camphor, etc.

Examples of the anti-inflammatory agent include allantoin, glycyrrhizinic acid and salt thereof, glycyrrhetinic acid and stearyl glycyrrhetinate, tranexamic acid, azulene, etc.

Examples of the skin care component (such as whitening agent, cell activator, rough skin improver, blood circulation promoter, skin astringent, antiseborrheic agent) include whitening agents, such as placenta extract, arbutin, glutathione, and saxifragaceae extract; cell activators, such as royal jelly, photosensitive element, cholesterol derivative, and young calf blood extract; rough skin improvers; blood circulation promoters, such as nonanoic acid vanillylamide, benzyl nicotinate, β-butoxyethyl nicotinate, capsaicin, zingerone, cantharides tincture, ichthammol, caffeine, tannic acid, α-borneol, nicotinic acid tocopherol, inositol hexanicotinate, cyclandelate, cinnarizine, tolazoline, acetylcholine, verapamil, cepharanthine, and γ-oryzanol; skin astringents, such as zinc oxide and tannic acid; antiseborrheic agents, such as sulfur and thianthol; etc.

Examples of the vitamin include vitamin A, such as vitamin A oil, retinol, retinol acetate, and retinol palmitate; vitamin B, including vitamin $B_2$, such as riboflavin, riboflavin butyrate, and flavin adenine nucleotide, vitamin $B_6$, such as pyridoxine hydrochloride salt, pyridoxine dioctanoate, and pyridoxine tripalmitate, vitamin $B_{12}$ and its derivative, and vitamin $B_{15}$ and its derivative; vitamin C, such as L-ascorbic acid, L-ascorbic acid dipalmitate ester, sodium L-ascorbic acid-2-sulfate, and dipotassium L-ascorbic acid phosphate diester; vitamin D, such as ergocalciferol and cholecalciferol; vitamin E, such as α-tocopherol, β-tocopherol, γ-tocopherol, dl-α-tocopherol acetate, dl-α-tocopherol nicotinate, and dl-α-tocopherol succinate; nicotinic acid, such as nicotinic acid, benzyl nicotinate, and nicotinic acid amide; vitamin H; vitamin P; pantothenic acid, such as calcium pantothenate, D-pantothenyl alcohol, pantothenyl ethyl ether, and acetyl pantothenyl ethyl ether; biotin; etc.

Examples of the amino acid include glycine, valine, leucine, isoleucine, serine, threonine, phenylalanine, arginine, lysine, aspartic acid, glutamic acid, cystine, cysteine, methionine, tryptophan, etc.
Examples of the nucleic acid include nucleic acids, such as deoxyribonucleic acid.
Examples of the hormone include estradiol, ethenyl estradiol, etc.
Examples of the inclusion compound include cyclodextrin, etc.
The inventive cosmetic includes cosmetics in which the cosmetic component(s) described above are blended, such as make-up cosmetics, including skin care cosmetic, milky lotion, cream, make-up foundation, concealer, white powder, liquid foundation, oil foundation, rouge, eye shadow, mascara, eye liner, eye blow, and lipstick; hair cosmetics, including shampoo, conditioner, treatment, and setting material; antiperspirant; UV-protective cosmetics, including sunscreen oil, sunscreen lotion, and sunscreen cream; etc.
Moreover, as the form of these cosmetics, various forms can be selected, such as liquid, milky lotion, cream, solid, paste, gel, powder, pressed, laminated, mousse, spray, and stick forms.
Further, as the type of these cosmetics, various types can be selected, such as water-base, oil-base, water-in-oil emulsion, oil-in-water emulsion, non-aqueous emulsion, and multi-emulsion, including W/O/W and O/W/O.

## EXAMPLE

Hereinafter, the present invention will be specifically described with reference to Production examples, Examples of the inventive cosmetic, and Comparative Examples. However, the present invention is not limited to the following Examples. Incidentally, unless otherwise especially noted, "%" described below means "mass%", and mass% of each component is expressed with the total mass in each example being taken as 100%. The viscosity is a value at 25°C.
Polyoxyalkylene group-containing organopolysiloxanes used in Production examples are to be illustrated. Note that the polyoxyalkylene group-containing organopolysiloxanes illustrated below can be synthesized by known methods. Examples thereof include those in Japanese Patent Application Nos. 2002-556651 and 2000-169265.
·Polyoxyalkylene group-containing organopolysiloxane 1 weight-average molecular weight: 8,000, HLB: 16

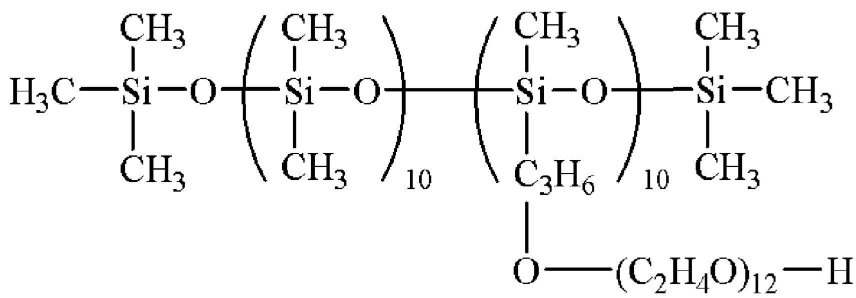

·Polyoxyalkylene group-containing organopolysiloxane 2 weight-average molecular weight: 4,000, HLB: 8.7

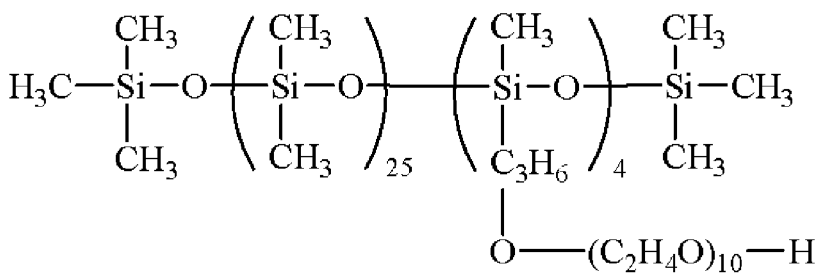

·Polyoxyalkylene group-containing organopolysiloxane 3 weight-average molecular weight: 210,000, HLB: 10

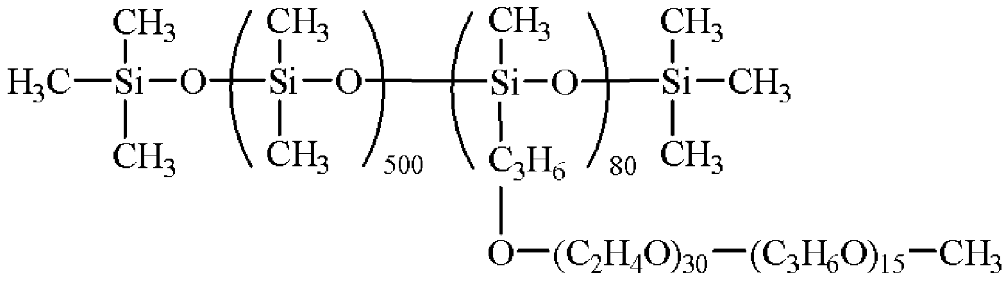

·Polyoxyalkylene group-containing organopolysiloxane 4 weight-average molecular weight: 14,000, HLB: 12.3

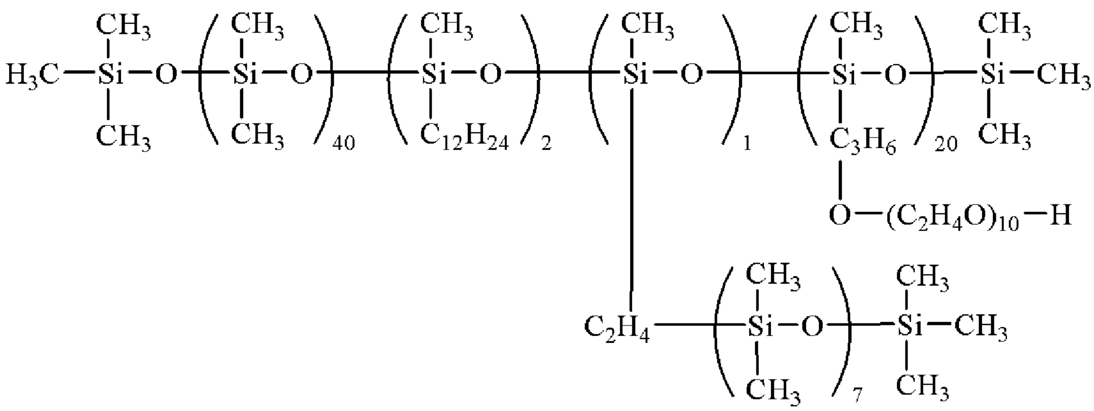

·Polyoxyalkylene group-containing organopolysiloxane 5 weight-average molecular weight: 600, HLB: 15

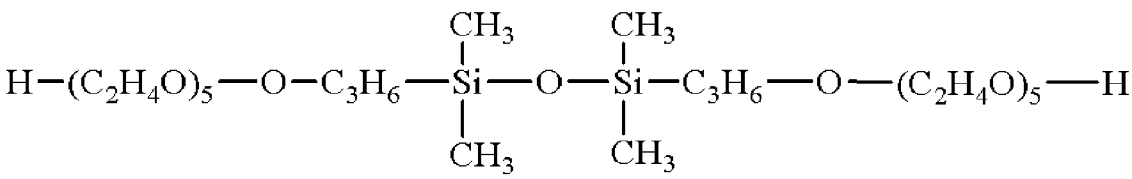

·Comparative polyoxyalkylene group-containing organopolysiloxane 1
weight-average molecular weight: 3,000, HLB: 6

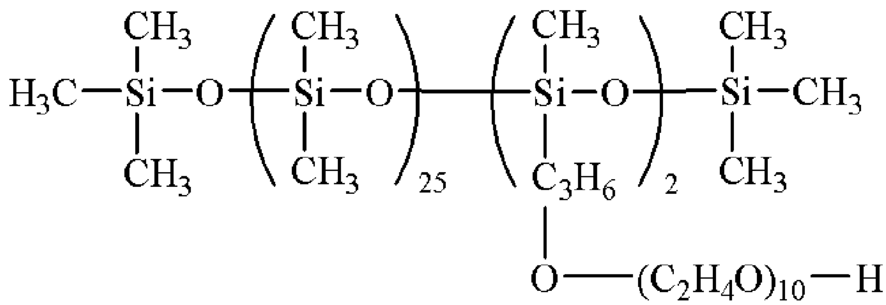

·Comparative polyoxyalkylene group-containing organopolysiloxane 2
weight-average molecular weight: 250, HLB: 3.5

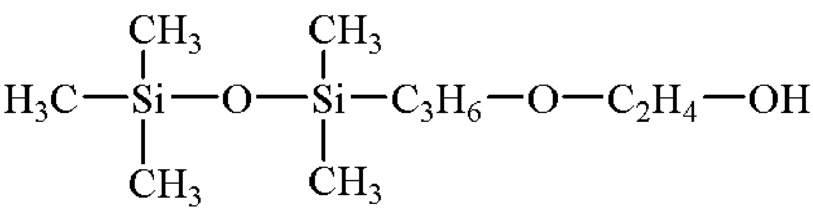

·Comparative polyoxyalkylene group-containing organopolysiloxane 3
weight-average molecular weight: 320,000, HLB: 6.4

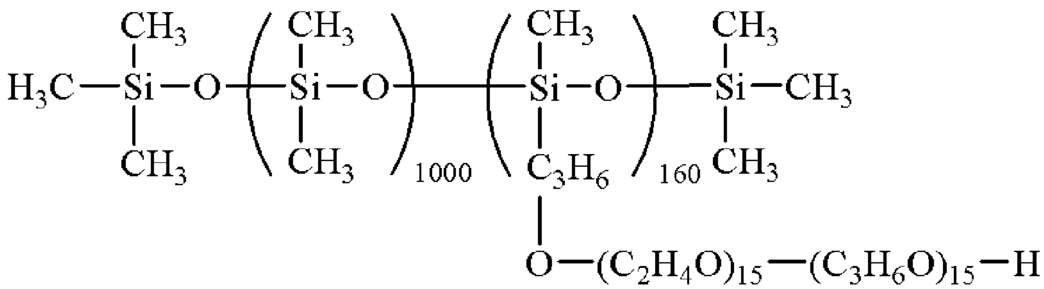

(Production examples 1 to 5), (Comparative production examples 1 to 3)

Production example 1

Into a bead mill, 100 g of (dimethicone/vinyl dimethicone) crosspolymer (average particle diameter: 10 μm), 3 g of Polyoxyalkylene group-containing organopolysiloxane 1, and 50 g of ethanol were added and stirred for 3 hours. Then, the mixture was dried at 110°C for 3 hours and pulverized. Thus, a white powder was obtained. This powder well dispersed in water, and hardly agglomerated over time.

Production example 2

Into a hammer mill, 100 g of (vinyl dimethicone/methicone silsesquioxane) crosspolymer (average particle diameter: 5 μm) and 4 g of Polyoxyalkylene group-containing organopolysiloxane 2 were added, stirred at 100°C for 3 hours, and pulverized. Thus, a white powder was obtained. This powder well dispersed in water, and hardly agglomerated over time.

Production example 3

Into a Nauta mixer, 100 g of polysilicone-22 (average particle diameter: 1 μm) and 2 g of Polyoxyalkylene group-containing organopolysiloxane 3 diluted with 10 g of ethanol were added, stirred at 100°C for 3 hours, and pulverized. Thus, a white powder was obtained. This powder well dispersed in water, and hardly agglomerated over time.

Production example 4

Into a hammer mill, 100 g of (vinyl dimethicone/methicone silsesquioxane) crosspolymer and 5 g of Polyoxyalkylene group-containing organopolysiloxane 4 diluted with 10 g of purified water were added, stirred at 100°C for 3 hours, and pulverized. Thus, a white powder was obtained. This powder well dispersed in water, and hardly agglomerated over time.

Production example 5

Into a hammer mill, 100 g of (vinyl dimethicone/methicone silsesquioxane) crosspolymer and 1 g of Polyoxyalkylene group-containing organopolysiloxane 5 diluted with 10 g of 2-propanol were added, stirred at 100°C for 3 hours, and pulverized. Thus, a white powder was obtained. This powder well dispersed in water, and hardly agglomerated over time.

Comparative production example 1

Into a bead mill, 100 g of (dimethicone/vinyl dimethicone) crosspolymer, 3 g of Comparative polyoxyalkylene group-containing organopolysiloxane 1, and 50 g of ethanol were added and stirred for 3 hours. Then, the mixture was dried at 110°C for 3 hours and pulverized. Thus, a white powder was obtained. This powder did not disperse in water, and agglomerated.

Comparative production example 2

Into a hammer mill, 100 g of (vinyl dimethicone/methicone silsesquioxane) crosspolymer and 4 g of polyglyceryl-6 oleate (HLB: 9) were added, stirred at 100°C for 3 hours, and pulverized. Thus, a white powder was obtained. This powder did not disperse in water, and agglomerated.

Comparative production example 3

Into a Nauta mixer, 100 g of polysilicone-22 and 2 g of sodium lauryl sulfate diluted with 10 g of purified water were added, stirred at 100°C for 3 hours, and pulverized. Thus, a white powder was obtained. This powder dispersed in water, but agglomerated over time.

Comparative production example 4

Into a bead mill, 100 g of (dimethicone/vinyl dimethicone) crosspolymer, 3 g of Comparative polyoxyalkylene group-containing organopolysiloxane 2, and 50 g of ethanol were added and stirred for 3 hours. Then, the mixture was dried at 110°C for 3 hours and pulverized. Thus, a white powder was obtained. This powder did not disperse in water, and agglomerated.

Comparative production example 5

Into a bead mill, 100 g of (dimethicone/vinyl dimethicone) crosspolymer, 3 g of Comparative polyoxyalkylene group-containing organopolysiloxane 3, and 50 g of ethanol were added and stirred for 3 hours. Then, the mixture was dried at 110°C for 3 hours and pulverized. Thus, a white powder was obtained. This powder dispersed in water, but agglomerated over time.

(Examples 1 to 5 and Comparative Examples 1 to 8)

(Evaluation 1)

Lotions shown in the following table were produced according to a conventional method to evaluate the usability.

[Sensory Evaluation Method]

Approximately 5 g of each lotion obtained according to the formulations shown in Table 1 below was applied to the skin, and subjected to sensory evaluations for spreadability upon application, uniformity, and stretchability. Note that the spreadability upon application refers to touch or application comfortability such as easiness (lightness) and smoothness when a cosmetic was applied and spread; and the stretchability refers to a length or area of the cosmetic physically spread. Moreover, regarding the dispersion stability, whether or not the powder agglomerated was visually evaluated based on the product appearance one week after the production. The result is shown by the following evaluation criteria according to the number of panelists who stated "effective".

[Evaluation Criteria]

A (excellent): 4 to 5 panelists stated effective
B (good): 3 panelists stated effective
C (fair): 2 panelists stated effective
D (poor): 1 or 0 panelists stated effective

[Table 1]

| | | Example | | | | | Comparative Example | | | | | | | |
|---|---|---|---|---|---|---|---|---|---|---|---|---|---|---|
| | | 1 | 2 | 3 | 4 | 5 | 1 | 2 | 3 | 4 | 5 | 6 | 7 | 8 |
| 1 | White powder obtained in Production example 1 | 5 | | | | | | | | | | | | |
| 2 | White powder obtained in Production example 2 | | 10 | | | | | | | | | | | |
| 3 | White powder obtained in Production example 3 | | | 2 | | | | | | | | | | |
| 4 | White powder obtained in Production example 4 | | | | 5 | | | | | | | | | |
| 5 | White powder obtained in Production example 5 | | | | | 5 | | | | | | | | |
| 6 | White powder obtained in Comparative production example 1 | | | | | | 5 | | | | | | | |
| 7 | White powder obtained in Comparative production example 2 | | | | | | | 10 | | | | | | |
| 8 | White powder obtained in Comparative production example 3 | | | | | | | | 2 | | | | | |
| 9 | White powder obtained in Comparative production example 4 | | | | | | | | | | | | 5 | |
| 10 | White powder obtained in Comparative production example 5 | | | | | | | | | | | | | 5 |
| 11 | (Vinyl dimethicone/ methicone silsesquioxane) crosspolymer | | | | | | | | | 10 | 10 | 10 | | |
| 12 | Dimethicone copolyol (HLB: 15) | | | | | | | | | 0.2 | | | | |
| 13 | Polyoxyalkylene group-containing organopolysiloxane 2 | | | | | | | | | | 0.2 | | | |
| 14 | Polyoxyalkylene group-containing organopolysiloxane 3 | | | | | | | | | | | 0.2 | | |
| 15 | 1.3-Butylene glycol | 5 | 5 | 5 | 5 | 5 | 5 | 5 | 5 | 5 | 5 | 5 | 5 | 5 |
| 16 | Concentrated glycerin | 8 | 8 | 8 | 8 | 8 | 8 | 8 | 8 | 8 | 8 | 8 | 8 | 8 |
| 17 | Dipropylene glycol | 6 | 6 | 6 | 6 | 6 | 6 | 6 | 6 | 6 | 6 | 6 | 6 | 6 |
| 18 | Sorbitol | 0.5 | 0.5 | 0.5 | 0.5 | 0.5 | 0.5 | 0.5 | 0.5 | 0.5 | 0.5 | 0.5 | 0.5 | 0.5 |
| 19 | Purified water | bala-nce | bala-nce | bala-nce | bala-nce | bala-nce | bala-nce | bala-nce | bala-nce | bala-nce | bala-nce | bala-nce | bala-nce | bala-nce |
| Evaluation | Dispersion stability after 1 week | A | A | B | A | B | C | D | C | D | D | D | D | C |
| | Spreadability upon application | A | A | A | B | A | B | C | C | C | C | C | D | D |
| | Uniformity upon application | B | A | A | A | A | C | D | C | C | C | C | C | C |
| | Stretchability | A | B | A | B | A | C | C | C | B | C | C | C | C |

The formulations were adjusted, so that the total of the components was 100.

As shown in Table 1, it was verified that quite excellent dispersibility, high dispersion stability, uniform cosmetic films, favorable spreadability upon application, and favorable stretchability are achieved by the lotions of Examples 1 to 5, which were obtained by using the inventive dispersible powders of the organopolysiloxane elastomer powders treated with the surface treatment agents each being an organopolysiloxane having a polyoxyalkylene group, a weight-average molecular weight of 300 to 300,000, and an HLB of 8 to 18.

In contrast, in Comparative Example 1 using the surface treatment agent which had an HLB lower than the lower

limit of the scope of the present invention, the dispersion stability, uniformity upon application, and stretchability were inferior.

Moreover, in Comparative Examples 2, 3 not using a polyoxyalkylene group-containing organopolysiloxane as the surface treatment agent, the dispersion stability, spreadability upon application, uniformity, and stretchability were inferior.

In Comparative Examples 4 to 6, in which the organopolysiloxane elastomer powders were not treated with a surface treatment agent, at least the dispersion stability, spreadability upon application, and uniformity were inferior.

In Comparative Examples 7, 8 using the surface treatment agents which had an HLB lower than the lower limit of the scope of the present invention and a weight-average molecular weight outside the scope of the present invention, the dispersion stability, spreadability upon application, uniformity, and stretchability were inferior.

Note that, although not shown in Table 1, it was impossible to prepare, as a surface treatment agent, a polyoxyalkylene group-containing organopolysiloxane having an HLB exceeding the upper limit of the scope of the present invention.

These revealed that the inventive dispersible powders are excellent in dispersibility, high in dispersion stability, capable of forming a uniform cosmetic film when blended in a cosmetic, and have favorable spreadability upon application, and favorable stretchability, adhesiveness, and feeling on use.

[Example 6]

O/W Sunscreen

<Preparation of Cosmetic>

A: Components (9) to (15) were homogeneously mixed at 85°C.
B: Components (1) to (6) were homogeneously mixed at 85°C.
C: At 80°C, the mixture B and components (7), (8) were added to the mixture A, emulsified, and gradually cooled. Thus, an O/W sunscreen was obtained.

| | Components | mass(%) |
|---|---|---|
| (1) | Polyglyceryl-10 laurate | 3.5 |
| (2) | Glyceryl stearate (SE) | 3 |
| (3) | Behenyl alcohol | 3 |
| (4) | Ethylhexyl methoxycinnamate | 5 |
| (5) | Isononyl isononanoate | 5 |
| (6) | Coco-caprylate/caprate | 5 |
| (7) | Silicone-treated fine-particle zinc oxide dispersion (Note 1) | 10 |
| (8) | Metallic-soap-treated fine-particle titanium oxide dispersion (Note 2) | 10 |
| (9) | Hydroxyethylcellulose | 0.2 |
| | 1,3-Butylene glycol | 10 |
| (11) | Ethanol | 6 |
| (10) (12) | Ethylhexylglycerin | 0.2 |
| (13) | Bisabolol | 0.2 |
| (14) | White powder of Production example 4 | 4 |
| (15) | Purified water | balance |
| | Total | 100% |

(Note 1) manufactured by Shin-Etsu Chemical Co., Ltd.; SPD-Z5
(Note 2) manufactured by Shin-Etsu Chemical Co., Ltd.; SPD-T5

It was verified that the obtained O/W sunscreen is excellent in feeling on use, applicability, and stability over time.

[Example 7]

Non-Aqueous Sunscreen

<Preparation of Cosmetic>

A: Components (1) to (5) were homogeneously mixed and dissolved.
B: Components (6) to (9) were homogeneously mixed.
C: The mixtures A and B were homogeneously mixed. Thus, a non-aqueous sunscreen was obtained.

| | Components | mass(%) |
|---|---|---|
| (1) | Diisopropyl sebacate | balance |
| (2) | Ethylhexyl methoxycinnamate | 7.5 |
| (3) | Bis-ethylhexyloxyphenol methoxyphenyl triazine | 1 |
| (4) | Stearyl glycyrrhetinate | 0.2 |
| (5) | BHT | 0.1 |
| (6) | Silicone/alkyl-modified, partially crosslinked polyglycerin-modified silicone composition (Note 1) | 20 |
| (7) | Phenyl-modified partially crosslinked dimethylpolysiloxane composition (Note 2) | 30 |
| (8) | Metallic-soap-treated fine-particle titanium oxide dispersion (Note 3) | 20 |
| (9) | White powder of Production example 2 | 4 |
| | Total | 100 |

(Note 1) manufactured by Shin-Etsu Chemical Co., Ltd.; KSG-850Z
(Note 2) manufactured by Shin-Etsu Chemical Co., Ltd.; KSG-18A
(Note 3) manufactured by Shin-Etsu Chemical Co., Ltd.; SPD-T5

It was verified that the obtained non-aqueous sunscreen is excellent in feeling on use, applicability, and stability over time.

[Example 8]

Powder Foundation

<Preparation of Cosmetic>

A: Components 1 to 3 were homogeneously mixed.
B: Components 4 to 12 were homogeneously mixed.
C: The mixture A was added to the mixture B, and homogeneously mixed using a Henschel mixer. The obtained powder was passed through a 100-mesh net, followed by molding in a metal dish using a mold. Thus, a powder foundation was obtained.

| | Components | mass(%) |
|---|---|---|
| 1. | 2-Ethylhexyl para-methoxycinnamate | 4 |
| 2. | Diphenylsiloxy phenyl trimethicone (Note 1) | 4.5 |
| 3. | Glyceryl triethylhexanoate | 1.5 |
| 4. | Barium sulfate | 10 |
| 5. | White powder of Production example 2 | 5 |
| 6. | Crosslinked dimethylpolysiloxane (Note 2) | 4 |
| 7. | Alkyl/silicone-branched silicone-treated mica (Note 3) | 30 |
| 8. | Alkyl/silicone-branched silicone-treated talc (Note 3) | 33.3 |
| 9. | Alkyl/silicone-branched silicone-treated titanium oxide (Note 4) | 6 |
| 10. | Alkyl/silicone-branched silicone-treated yellow iron oxide (Note 4) | 1.0 |
| 11. | Alkyl/silicone-branched silicone-treated red iron oxide (Note 4) | 0.5 |
| 12. | Alkyl/silicone-branched silicone-treated black iron oxide (Note 4) | 0.2 |

(continued)

| Components | mass(%) |
|---|---|
| Total | 100.0% |

(Note 1) manufactured by Shin-Etsu Chemical Co., Ltd.; KF-56A
(Note 2) manufactured by Shin-Etsu Chemical Co., Ltd.; KSG-15
(Note 3) manufactured by Shin-Etsu Chemical Co., Ltd.; treated with KF-9909
(Note 4) manufactured by Shin-Etsu Chemical Co., Ltd.; KTP-09-Series

The obtained powder foundation was fine and spread lightly. The foundation was excellent in adhesion.

[Example 9]

O/W Cream

<Preparation of Cosmetic>

A: Components (4) to (11) were mixed.
B: Components (1) to (3) were mixed, added to the mixture A, and emulsified by stirring.

| | Components | mass(%) |
|---|---|---|
| (1) | Crosslinked dimethylpolysiloxane (Note | 8. |
| (2) | Glyceryl triethylhexanoate | 5.0 |
| (3) | Alkyl/silicone-branched silicone-treated titanium oxide (Note 2) | 1.0 |
| (4) | White powder of Production example 2 | 2.0 |
| (5) | Dipropylene glycol | 7.0 |
| (6) | Glycerin | 5.0 |
| (7) | Methylcellulose (2% aqueous solution) (Note 3) | 7.0 |
| (8) | Polyacryl amide-based emulsifier (Note 4) | 2.0 |
| (9) | Preservative | 0.1 |
| (10) | Fragrance | 0.2 |
| (11) | Purified water | balance |
| | Total | 100.0% |

(Note 1) manufactured by Shin-Etsu Chemical Co., Ltd.; KSG-16
(Note 2) manufactured by Shin-Etsu Chemical Co., Ltd.; KTP-09W
(Note 3) manufactured by Shin-Etsu Chemical Co., Ltd.; METOLOSE SM-4000
(Note 4) manufactured by SEPPIC S.A.; SEPIGEL 305

The obtained O/W cream was spread smoothly without sticking, and had fresh feeling on use and also excellent stability over time.

[Example 10]

Non-Aqueous Concealer

<Preparation of Cosmetic>

A: Components (7) to (12) were prepared into a paste with a three-roll mill.
B: The components in A and (1) to (6) were homogeneously mixed. Thus, a non-aqueous concealer was obtained.

| | Components | mass (%) |
|---|---|---|
| (1) | Phenyl-modified silicone composite powder (Note 1) | 20 |

(continued)

| | Components | mass (%) |
|---|---|---|
| (2) | White powder of Production example 2 | 8 |
| (3) | Crosslinked dimethylpolysiloxane (Note 2) | 6 |
| (4) | Dimethylpolysiloxane (6cs) | 40 |
| (5) | Methylphenylpolysiloxane (Note 3) | 5 |
| (6) | Decamethylcyclopentasiloxane | 12.9 |
| (7) | Glyceryl triethylhexanoate | 2.2 |
| (8) | Silicone-branched polyether-modified silicone (Note 4) | 0.5 |
| (9) | Alkyl/silicone-branched silicone-treated titanium oxide (Note 5) | 5 |
| (10) | Alkyl/silicone-branched silicone-treated yellow iron oxide (Note 5) | 0.25 |
| (11) | Alkyl/silicone-branched silicone-treated red iron oxide (Note 5) | 0.1 |
| (12) | Alkyl/silicone-branched silicone-treated black iron oxide (Note 5) | 0.05 |
| | Total | 100.0% |

(Note 1) manufactured by Shin-Etsu Chemical Co., Ltd.; KSP-300
(Note 2) manufactured by Shin-Etsu Chemical Co., Ltd.; KSG-16
(Note 3) manufactured by Shin-Etsu Chemical Co., Ltd.; KF-56A
(Note 4) manufactured by Shin-Etsu Chemical Co., Ltd.; KF-6028P
(Note 5) manufactured by Shin-Etsu Chemical Co., Ltd.; KTP-09-Series

The stretchability and adhesion of the powder in the obtained non-aqueous concealer were favorable. The non-aqueous concealer had light touch and favorable stability over time.

[Example 11]

Milky Lotion

<Preparation of Cosmetic>

Components (1) to (9) were homogeneously mixed.

| | Components | mass (%) |
|---|---|---|
| (1) | Ethanol | 5.00 |
| (2) | Glycerin | 4.00 |
| (3) | 1,3-Butylene glycol | 3.00 |
| (4) | Purified water | balance |
| (5) | Carboxy vinyl polymer (1% aqueous solution) | 15.00 |
| (6) | Sodium hydroxide | 0.05 |
| (7) | Xanthan gum (2% aqueous solution) | 10.00 |
| (8) | Disodium edetate | 0.05 |
| (9) | White powder of Production example 1 | 10.00 |
| | Total | 100.00 |

The obtained milky lotion was spread smoothly, and had favorable smoothness, fresh feeling on use, and excellent stability over time, too.

[Example 12]

Hair Conditioner

<Preparation of Cosmetic>

A: Components (1) to (6) were heated and mixed, and further component (10) was added, mixed, and cooled.
B: Components (7) to (9) were mixed, added to the mixture A, and thoroughly mixed.

| | Components | mass (%) |
|---|---|---|
| | Cetostearyl alcohol | 2 |
| (2) | Cetyl 2-ethylhexanoate | 3 |
| (3) | Behentrimonium chloride | 1 |
| (1) (4) | Paraben (preservative) | q. s. |
| (5) | Propylene glycol | 5 |
| (6) | Methylphenylpolysiloxane (Note 1) | 1 |
| (7) | Hydroxyethylcellulose (1% aqueous solution) | 10 |
| (8) | White powder of Production example 2 | 5 |
| (9) | Fragrance | q. s. |
| (10) | Purified water | balance |
| | Total | 100.0% |
| (Note 1) manufactured by Shin-Etsu Chemical Co., Ltd.; KF-56A | | |

The obtained hair conditioner did not cause the hair to be tangled when rinsed off. The rinsed hair had smooth finish.

[Example 13]

O/W Cosmetic Foundation

<Preparation of Cosmetic>

Components (1) to (8) were homogeneously mixed.

| | Components | mass (%) |
|---|---|---|
| (1) | White powder of Production example 1 | 5 |
| (2) | Ethanol | 5 |
| (3) | 1,3-Butylene glycol | 6 |
| (4) | Carboxy vinyl polymer (2% aqueous solution) (Note 1) | 20 |
| (5) | Xanthan gum (2% aqueous solution) | 12 |
| (6) | Sodium chloride | 0.01 |
| (7) | Preservative | q. s. |
| (8) | Purified water | balance |
| | Total | 100. 0% |
| (Note 1) manufactured by Clariant; ARISTOFLEX AVC | | |

The obtained O/W cosmetic foundation was spread well, and had a beautifying effect by making indistinct the unevenness of the skin. The skin coated therewith had favorable smoothness. Moreover, the stability over time was also favorable.

[Example 14]

Lip Cream

<Preparation of Cosmetic>

A: A part of component (2) was mixed with component (9), and dispersed using a roller mill. Then, the obtained dispersion

was mixed with components (1) to (5) while being heated.
B: Components (6) to (8) were heated, added to the mixture obtained in "A", emulsified, and then cooled.
C: Component (10) was added to the emulsified product obtained in "B". Thereby, a lip cream was obtained.

| | Components | mass (%) |
|---|---|---|
| (1) | Dextrin palmitate/ethylhexanoate (Note 1) | 9 |
| (2) | Glyceryl triethylhexanoate | 5 |
| (3) | Silicone acrylate (Note 2) | 5 |
| (4) | Alkyl-modified branched-polyglycerin-modified silicone (Note 3) | 2 |
| (5) | Decamethylcyclopentasiloxane | 45 |
| (6) | 1,3-Butylene glycol | 5 |
| (7) | White powder of Production example 3 | 3 |
| (8) | Purified water | 16 |
| (9) | iron oxide | q.s. |
| (10) | Mica | q. s. |
| | Total | 100.0% |

(Note 1) manufactured by Chiba Flour Milling Co., Ltd.; Rheopearl TT
(Note 2) manufactured by Shin-Etsu Chemical Co., Ltd.; KP-561P
(Note 3) manufactured by Shin-Etsu Chemical Co., Ltd.; KF-6105

The obtained lip cream had light spreadability and was free from stickiness and oily feeling, and a highly durable film was formed on the lip. Moreover, the lip cream did not cause bitter or unpleasant sensation when applied, and was easy to use.

## Claims

**1.** A dispersible powder comprising an organopolysiloxane elastomer powder treated with a surface treatment agent, wherein

the surface treatment agent is an organopolysiloxane having a polyoxyalkylene group, a weight-average molecular weight of 300 to 300,000, an HLB of 8 to 18, and
the organopolysiloxane having a polyoxyalkylene group has a structure shown by the following general formula (1),

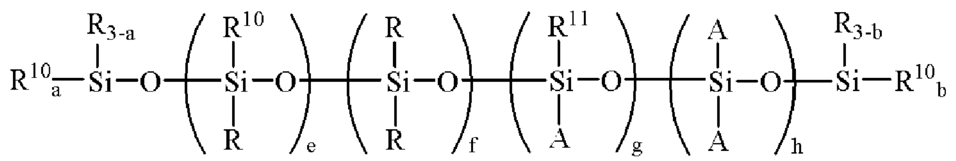
(1)

in the formula (1), each R independently represents an alkyl group having 1 to 16 carbon atoms, a fluoroalkyl group having 1 to 10 carbon atoms, or an aryl group having 6 carbon atoms;
each $R^{10}$ represents a polyoxyalkylene group shown by the following general formula (2),

$$X\text{-}O\text{-}(C_2H_4O)_s\text{-}(C_3H_6O)_t\text{-}R^{12} \qquad (2)$$

in the formula (2), X represents a divalent hydrocarbon group having 1 to 10 carbon atoms, "s" represents an integer of 1 to 100, "t" represents an integer of 0 to 50, and $R^{12}$ represents a hydrogen atom, a monovalent hydrocarbon group having 1 to 6 carbon atoms, or a monovalent acyl group having 1 to 6 carbon atoms;
$R^{11}$ represents an organic group selected from $R^{10}$ or R; and
"A" represents an organopolysiloxane segment shown by the following general formula (3) or (4),

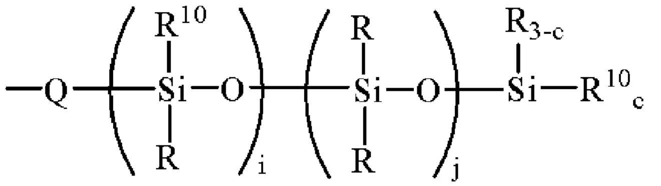 (3)

$-X-Si(OSi(CH_3)_3)_3$ (4)

in the general formula (3), R and $R^{10}$ are as defined above, and Q represents an oxygen atom or a divalent hydrocarbon group having 1 to 3 carbon atoms; in the general formula (4), X is as defined above, and

in the general formulae (1) and (3), "a", "b", and "c" each independently represent an integer of 0 to 3, "e" represents an integer of 0 to 500, "f" represents an integer of 0 to 50,000, "g" represents an integer of 0 or 1, "h" represents an integer of 0 or 1, "i" represents an integer of 0 to 500, and "j" represents an integer of 0 to 10,000, provided that when $R^{11}$ is $R^{10}$, $1 \leq a+b+c+e+g+i$, and when $R^{11}$ is R, $1 \leq a+b+c+e+i$; and at least one $R^{10}$ is present in the general formula (1).

**2.** A cosmetic comprising the dispersible powder according to claim 1.

**3.** The cosmetic according to claim 2, further comprising water.

**4.** The cosmetic according to claim 3, further comprising a hydrophilic substance selected from propylene glycol, trimethylene glycol, dipropylene glycol, 1,3-butylene glycol, methylpropanediol, pentylene glycol, glycerin, diglycerin, ethylhexylglycerin, triglycerin, polyglycerin, and ethanol.

## Patentansprüche

**1.** Dispergierbares Pulver, das ein mit einem Oberflächenbehandlungsmittel behandeltes Organopolysiloxan-Elastomerpulver umfasst, wobei

das Oberflächenbehandlungsmittel ein Organopolysiloxan mit einer Polyoxyalkylengruppe, einem gewichtsmittleren Molekulargewicht von 300 bis 300.000, einem HLB von 8 bis 18 ist und

das Organopolysiloxan mit einer Polyoxyalkylengruppe eine Struktur aufweist, die durch die folgende allgemeine Formel (1) dargestellt wird,

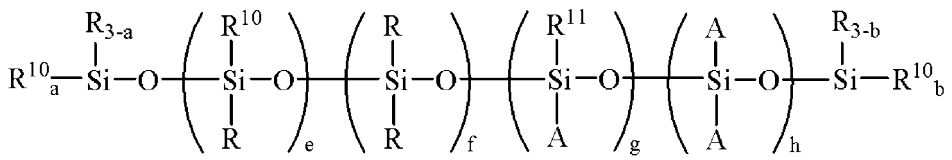 (1)

wobei in der Formel (1) jedes R unabhängig voneinander eine Alkylgruppe mit 1 bis 16 Kohlenstoffatomen, eine Fluoralkylgruppe mit 1 bis 10 Kohlenstoffatomen oder eine Arylgruppe mit 6 Kohlenstoffatomen repräsentiert; jedes $R^{10}$ eine Polyoxyalkylengruppe der folgenden allgemeinen Formel (2) repräsentiert,

$-X-O^{-}(C_2H_4O)_s-(C_3H_6O)_t-R^{12}$ (2)

wobei in der Formel (2) X eine zweiwertige Kohlenwasserstoffgruppe mit 1 bis 10 Kohlenstoffatomen repräsentiert, "s" eine ganze Zahl von 1 bis 100, "t" steht für eine ganze Zahl von 0 bis 50 repräsentiert und $R^{12}$ ein Wasserstoffatom, eine einwertige Kohlenwasserstoffgruppe mit 1 bis 6 Kohlenstoffatomen oder eine einwertige Acylgruppe mit 1 bis 6 Kohlenstoffatomen repräsentiert;

$R^{11}$ eine organische Gruppe repräsentiert, die aus $R^{19}$ oder R ausgewählt ist; und

"A" ein Organopolysiloxansegment repräsentiert, das durch die folgende allgemeine Formel (3) oder (4) dargestellt wird,

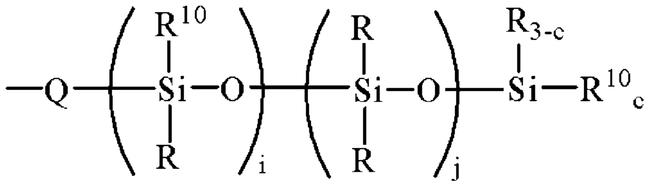

(3)

$-X--Si(OSi(CH_3)_3)_3$ (4)

wobei in der allgemeinen Formel (3) R und $R^{10}$ wie oben definiert sind, und Q ein Sauerstoffatom oder eine zweiwertige Kohlenwasserstoffgruppe mit 1 bis 3 Kohlenstoffatomen repräsentiert; wobei in der allgemeinen Formel (4) X wie oben definiert ist, und

wobei in den allgemeinen Formeln (1) und (3) "a", "b" und "c" jeweils unabhängig voneinander eine ganze Zahl von 0 bis 3 repräsentieren, "e" eine ganze Zahl von 0 bis 500 repräsentiert, "f" eine ganze Zahl von 0 bis 50.000 repräsentiert, "g" eine ganze Zahl von 0 oder 1 repräsentiert, "h" eine ganze Zahl von 0 oder 1 repräsentiert, "i" eine ganze Zahl von 0 bis 500 repräsentiert und "j" eine ganze Zahl von 0 bis 10.000 repräsentiert, vorausgesetzt, dass, wenn $R^{11}$ $R^{10}$ ist, $1 \leq a+b+c+e+g+i$, und wenn $R^{11}$ R ist, $1 \leq a+b+c+e+i$ gilt; und mindestens ein $R^{10}$ in der allgemeinen Formel (1) vorhanden ist.

2. Kosmetikum, das das dispergierbare Pulver gemäß Anspruch 1 umfasst.

3. Kosmetikum gemäß Anspruch 2, das zusätzlich Wasser umfasst.

4. Kosmetikum gemäß Anspruch 3, das ferner eine hydrophile Substanz umfasst, die aus Propylenglykol, Trimethylenglykol, Dipropylenglykol, 1,3-Butylenglykol, Methylpropandiol, Pentylenglykol, Glycerin, Diglycerin, Ethylhexylglycerin, Triglycerin, Polyglycerin und Ethanol ausgewählt ist.

## Revendications

1. Poudre dispersible comprenant une poudre d'élastomère organopolysiloxane traitée avec un agent de traitement de surface, dans laquelle

l'agent de traitement de surface est un organopolysiloxane comportant un groupe polyoxyalkylène, un poids moléculaire moyen en poids compris entre 300 et 300 000, un HLB compris entre 8 et 18, et l'organopolysiloxane comportant un groupe polyoxyalkylène a une structure représentée par la formule générale (1) suivante,

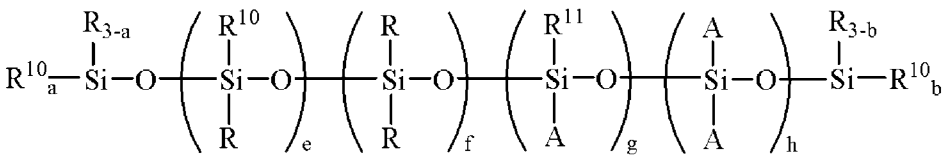

(1)

dans la formule (1), chaque R représente indépendamment un groupe alkyle ayant 1 à 16 atomes de carbone, un groupe fluoroalkyle ayant 1 à 10 atomes de carbone ou un groupe aryle ayant 6 atomes de carbone; chaque $R^{10}$ représente un groupe polyoxyalkylène représenté par la formule générale (2) suivante,

$-X-O(C_2H_4O)_s-(C_3H_6O)_t-R^{12}$ (2)

dans la formule (2), X représente un groupe hydrocarbure divalent ayant 1 à 10 atomes de carbone, « s » représente un nombre entier de 1 à 100, « t » représente un nombre entier de 0 à 50, et $R^{12}$ représente un atome d'hydrogène, un groupe hydrocarbone monovalent ayant 1 à 6 atomes de carbone, ou un groupe acyle monovalent ayant 1 à 6 atomes de carbone;

$R^{11}$ représente un groupe organique choisi parmi $R^{10}$ ou R; et

« A » représente un segment organopolysiloxane représenté par la formule générale (3) ou (4) suivante,

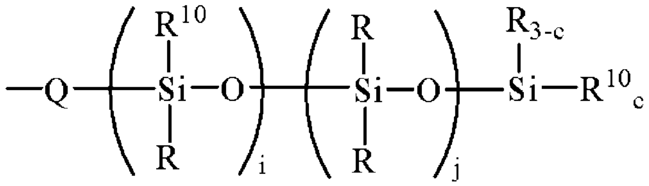

(3)

$$-X-Si(OSi(CH_3)_3)_3 \qquad (4)$$

dans la formule générale (3), R et $R^{10}$ sont tels que définis dans la description, et Q représente un atome d'oxygène ou un groupe hydrocarbone divalent ayant 1 à 3 atomes de carbone; dans la formule générale (4), X est tel que défini dans la description, et

dans les formules générales (1) et (3), « a », « b » et « c » représentent chacun indépendamment un nombre entier compris entre 0 et 3, « e » représente un nombre entier compris entre 0 et 500, « f » représente un nombre entier compris entre 0 et 50 000, « g » représente un nombre entier égal à 0 ou 1, « h » représente un nombre entier compris entre 0 et 1, « i » représente un nombre entier compris entre 0 et 500, et « j » représente un nombre entier compris entre 0 et 10 000, à condition que lorsque $R^{11}$ est $R^{10}$, $1 \leq a+b+c+e+g+i$, et lorsque $R^{11}$ est R, $1 \leq a+b+c+e+i$; et au moins un $R^{10}$ est présent dans la formule générale (1).

**2.** Produit cosmétique comprenant la poudre dispersible selon la revendication 1.

**3.** Produit cosmétique selon la revendication 2, comprenant en outre de l'eau.

**4.** Produit cosmétique selon la revendication 3, comprenant en outre une substance hydrophile choisie parmi le propylène glycol, le triméthylène glycol, le dipropylène glycol, le 1,3-butylène glycol, le méthylpropanediol, le pentylène glycol, la glycérine, la diglycérine, l'éthylhexylglycérine, la triglycérine, la polyglycérine et l'éthanol.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2001007672 A1, MIURA YOSHIMASA
- US 20010712 A1
- JP 1266141 A
- JP 2003026958 A
- JP 2001220319 A
- JP 2001114649 A
- US 2001007672 A
- JP 2010030954 A
- JP 2004300057 A
- JP 2015086150 A
- JP 2013139400 A
- JP 1925781 B
- JP 1932769 B
- WO 0324413 A1
- JP 2009185296 A
- JP 2631772 B
- JP H09136813 A
- JP 2001342255 A
- WO 0320828 A1
- JP 2002556651 A
- JP 2000169265 A